# EUROPÄISCHE PATENTSCHRIFT

(11) **EP 2 293 774 B1**
(45) Veröffentlichungstag und Bekanntmachung des Hinweises auf die Patenterteilung: **16.01.2019**
(21) Anmeldenummer: 09765501.3
(22) Anmeldetag: 25.05.2009
(51) Int. Cl.: A61K 9/20, A61K 47/26

(54) **DIREKT VERPRESSBARE POLYOLKOMBINATION**
DIRECTLY EXTRUDABLE POLYOL COMBINATION
COMBINAISON DE POLYOLS DIRECTEMENT COMPRESSIBLE

(30) Priorität: 20.06.2008 EP 08011231; 11.08.2008 EP 08014288
(43) Veröffentlichungstag der Anmeldung: 16.03.2011
(73) Patentinhaber: Merck Patent GmbH, 64293 Darmstadt (DE)
(72) Erfinder: HAMM, Walter, 64331 Weiterstadt (DE); KLEINWAECHTER, Daniela, 64289 Darmstadt (DE); MODDELMOG, Guenter, 64354 Reinheim (DE)
(86) Internationale Anmeldenummer: PCT/EP2009/003676
(87) Internationale Veröffentlichungsnummer: WO 2009/152921

(56) Entgegenhaltungen:
- DD-A3- 277 176
- DE-A1- 4 439 858
- DE-A1- 19 615 418
- DE-A1- 19 617 487
- US-A- 5 158 789

## Beschreibung

Die vorliegende Erfindung betrifft die Herstellung eines neuen Trägermaterials zur Herstellung von wirkstoff- und/oder aromastoffhaltigen Tablettenformulierungen, sowie die Verwendung dieses neuen Trägermaterials, welches direkt verpressbar ist und aus einer Polyolkombination besteht. Weiterhin schließt die vorliegende Erfindung aus diesem Trägermaterial hergestellte wirkstoffhaltige Tabletten ein.

### Stand der Technik

Tabletten sind einzeldosierte feste Arzneiformen die unter Pressdruck aus Pulvern oder Granulaten auf Tablettenpressen gefertigt werden.

Sehr viele Wirkstoffe können tablettiert werden. Einige dieser Wirkstoffe können direkt tablettiert werden, das heißt ohne weitere Verarbeitung des Pulvers oder Pulvergemisches (Direkttablettierung), während die meisten Wirkstoffe erst über die Zwischenstufe eines Granulats zu Tabletten gepresst werden können. In aller Regel werden neben dem eigentlichen Wirkstoff zusätzliche Hilfsstoffe eingearbeitet, um sowohl das Verhalten der zu tablettierenden Mischungen zu verbessern als auch die Eigenschaften der hergestellten Tabletten den gewünschten Anwendungsanforderungen anzupassen.

Unter Direkttablettierung versteht man das Verpressen der Wirkstoffpulver oder Wirkstoffgemische mit oder ohne Zusatz von Hilfsstoffen ohne weitere Vorbehandlung zu Tabletten. Sie ist damit einfach und billig im industriellen Maßstab durchzuführen. Es weisen jedoch nur wenige Substanzen die zur Direkttablettierung notwendigen Eigenschaften auf. Insbesondere die Fließfähigkeit der einzusetzenden Füllstoffe oder Trägermaterialien und insbesondere die Bindungskräfte zwischen den Partikeln der Mischungen liegen oft nicht in dem gewünschten Bereich. Während die Fließfähigkeit zu einer gleichmäßigen Dosierung während der Tablettierung beiträgt, sind die Bindekräfte für die durch das Verpressen zu erzielende Tablettenhärte und Abriebfestigkeit verantwortlich.

Als geeignete Zusätze, welche die Fließeigenschaften verbessern, sind u. a. hochdisperses Siliziumdioxid und Talkum bekannt. Siliziumdioxid besitzt eine hohe spezifische Oberfläche und kann bis zu 40% Wasser aufnehmen, ohne seine Fließfähigkeit zu verlieren.

Zugesetzte Binde- und Sprengmittel (Zerfallsmittel) wiederum verbessern das Verpressen zu haltbaren Tabletten und das spätere Zerfallen der Tabletten im Magen-Darm-Trakt:
Die Zugabe von Cellulose, insbesondere von mikrokristalliner Cellulose oder mikrofeiner Cellulose, führt zu bruchfesten Tabletten, verschlechtert aber die Fließeigenschaften der eingesetzten Pulvermischung. Durch das Quellvermögen der Pulvercellulose wird eine kurze Zerfallszeit erzielt. Weitere geeignete Bindemittel für die Tablettenherstellung sind die Cellulosederivate HPMC und HPC, sowie die Polyvinylpyrrolidone (PVP - Kollidone). Es handelt sich dabei um sehr effektive Feuchtbindemittel. Zu den Trockenbindemitteln, die zur Granulation ohne Zusatz von Wasser eingesetzt werden können, zählen wiederum die HPC-Varianten, sowie die Copovidone.

In der Praxis ist es für den Fachmann wünschenswert, möglichst wenige dieser zusätzlichen Tablettierhilfsstoffe einsetzen zu müssen und trotzdem gute Tablettierergebnisse zu erzielen.

So ist es seit längerem bekannt, zur Herstellung von Tablettenformulierungen geeignete Polyole einzeln oder im Gemisch einzusetzen. Je nachdem welche Methode eingesetzt wurde, um diese Trägermaterialien in trockener, pulverförmiger Form herzustellen, lassen sich nach Vermischen mit den pharmazeutisch aktiven Wirkstoffen durch Verpressen direkt Tabletten erhalten. Feuchte oder trockene Granulierungsschritte werden dadurch überflüssig.

Zu der Gruppe der zu diesem Zweck einsetzbaren Polyole zählen Zuckeraustauschstoffe, wie Sorbit, Mannit, Xylit, Isomalt, Maltit und Lactit.

In US 5,158,789 A (Duross James W) werden beispielsweise PolyolZusammensetzungen für die Herstellung von Tabletten oder Kaugummi beschrieben, die durch Schmelzkristallisation erhalten werden. Hierbei werden Sorbit und Xylit jeweils vorgetrocknet, so dass sie einen Wassergehalt von weniger als 3 %, vorzugweise weniger als 1 %, aufweisen, und in unterschiedlichen Gewichtsverhältnissen im Bereich von 75 : 25 bis 99 : 1 in der Schmelze miteinander in einem aufwändigen Verfahren unter Rühren verschmolzen, abgekühlt und gegebenenfalls extrudiert. Das erhaltene Produkt wird anschließend vermahlen und gegebenenfalls gesiebt. Beim Schmelzen ist darauf zu achten, dass die Temperatur nicht zu hoch eingestellt wird, da in diesem Fall die Auskristallisation erschwert ist. Weiterhin muss während der Kristallisation darauf geachtet werden, dass aus der Umgebung möglichst keine Feuchtigkeit mit dem Produkt in Kontakt kommt, um den Feuchtigkeitsgehalt des Produkts niedrig zu halten.

Gute Eigenschaften zeigen die oben genannten Polyole in diesem Zusammenhang, wenn sie vorab in besonderer Weise einer Sprühtrocknung aus einer wässrigen Lösung unterzogen worden sind, oder gegebenenfalls nach erfolgter Sprüh- oder Co-Sprühtrocknung bis auf eine bestimmte Restfeuchte einer anschließenden Wirbelschichtgranulation unterzogen worden sind. Die Herstellung solcher sprühgetrockneten Polyolqualitäten ist beispielsweise in EP 0738 252 B1, bzw. in DE 44 39858 A1, oder EP 0904059 B1 beschrieben. In dem Patent EP 0738 252 B1 (auch veröffentlicht in der korrespondierenden Anmeldung DE 4439 858 A1 (Merck Patent GmbH)) werden sprühgetrocknete Polyolzusammensetzungen beschrieben, worin Sorbit in Kombination mit einem oder zwei weiteren Polyolen sprühgetrocknet wird. Hierin kann ein Verhältnis von Sorbit zu Xylit in einem Bereich von 50 : 50 bis 99 : 1, insbesondere zwischen 65 : 35 bis 98 : 2 vorliegen. Vorzugsweise bestehen die beschriebenen Polyolzusammensetzungen zu 65 bis 94 Teilen (bzw. %) aus Sorbit und einem oder zwei weiteren Polyol(en), wobei in der Zusammensetzung weniger als 10 Gew.-% Mannit enthalten sein dürfen. Die Variation des Verhältnisses von Sorbit zu Xylit oder von Sorbit zu Xylit und einem weiteren Polyol kann hier also in einem sehr weiten Bereich erfolgen.

In DE 196 15 418 (Merck Patent GmbH) dagegen wird eine Zusammensetzung beschrieben, die mindestens zu 80 Gew.-% aus einem nicht-hygroskopischen Polyol besteht. Vorzugsweise wird als nichthygroskopisches Polyol Mannit mit einem weiteren Polyol und gegebenenfalls einem Bindemittel co-sprühgetrocknet oder cosprühgranuliert, um Tablettierbarkeit und Bindekapazität von Mannit zu verbessern und um ein alternatives Trägermaterial zu Sorbit zu erhalten, das gleichzeitig eine geringere Hygroskopizität als Sorbit aufweist.

Während Mannit, Lactit, Isomalt und Xylit üblicherweise gering hygroskopisch sind, und schlechtes Tablettierverhalten zeigen, was sich in geringer Tablettenhärte, Deckeln und hohem Abrieb der Tabletten bemerkbar macht, werden jedoch bei ihrer Verwendung hohe Tablettenhärten erzielt, wenn sie in geeigneter Weise sprüh- oder co-sprühgetrocknet werden.

Nachteil dieser sprüh- oder co-sprühgetrockneten Polyolqualitäten ist, dass sie bei der erhaltenen Kornstruktur eine verhältnismäßig geringe Schüttdichte zeigen. Beispielsweise wurde gefunden, dass ein entsprechendes Trägermaterial nach Sprühtrocknung nur eine Schüttdichte von ca. 35 g/100 ml aufweist und es bei Anwendung insbesondere höherer Presskräfte in den gängigen Tablettierungsanlagen nur zu Tabletten mit verhältnismäßig geringem Gewicht verpreßbar ist. So lässt sich z.B. ein Material solch geringer Schüttdichte nur zu rundenTabletten (bei einem Tablettendurch- messer von 11 mm) mit einem Gewicht von etwa 400 mg statt zu solchen mit einem geforderten Gewicht von 500 mg verpressen. Dieses hat auch den Nachteil, dass sich bei Verwendung eines solchen Trägermaterials Wirkstoffe schlechter dosieren lassen. Somit sind diese Trägermaterialien mit entsprechenden physikalischen Gemischen, bestehend aus Sorbit und Xylit nicht vergleichbar.

### Aufgabenstellung

Aufgabe der vorliegenden Erfindung ist es daher, ein Verfahren zur Verfügung zu stellen, durch das eine Tablettenmatrix erhalten wird, die aufgrund ihrer galenischen Eigenschaften zur Direkttablettierung geeignet ist und dabei über ein ausreichend hohes Schüttgewicht verfügt. Eine weitere Aufgabe besteht darin, eine tablettierbare Ausgangsmischung für wirkstoffhaltige Tablettenformulierungen zur Verfügung zu stellen, die möglichst wenige, bzw. keine zusätzlichen Tablettierhilfsmittel enthält, und die bereits bei relativ niedrigen Presskräften Tabletten mit hoher Härte liefert. Gleichzeitig ist es wünschenswert, entsprechende Tabletten herzustellen, deren Auflösungszeiten möglichst kurz sind, obwohl keine Binde- und Sprengmittel (Zerfallsmittel) enthalten sind, und deren Geschmack darüber hinaus angenehm süß und kühlend ist.

### Lösung der Aufgabe

Die Aufgabe der vorliegenden Erfindung wird gelöst durch die Bereitstellung einer direktverpreßbaren Kombination als Trägermaterial für die Herstellung von Wirkstoff- und/oder aromastoffhaltigen Tablettenformulierungen, bestehend aus einer durch kontinuierliche Cosprühgranulierung oder Cosprühtrocknung erhaltenen Mischung von Sorbit und Xylit im Gewichtsverhältnis von 7 : 3 mit einem Wassergehalt < 1 Gew.-%. Dieses cosprühgetrocknete oder cosprühgranulierte Material zeichnet sich bevorzugt dadurch aus, dass es eine Schüttdichte von 0,39 - 0,43 g/ml, einen Fließwinkel von 32,9 - 35,5° und eine Stampfdichte von 0,52 - 0,57g/ml besitzt. Weiterhin weist es bevorzugt eine spezifische Oberfläche gemäß BET-Verfahren im Bereich von 1,01 - 1,16 m²/g auf. Mindestens 88 Gew.-% dieses Materials weisen eine Kornverteilung der Partikeldurchmesser im Bereich von 50 - 500 µm auf, wobei der Anteil mit Durchmessern <50µm und > 500 µm jeweils weniger als 6 Gew.-% ausmachen. Die Partikelgrößenverteilung dieses Materials wird durch Trockensiebung über einen Siebturrm mit einem Siebdurchmesser von 200 mm ermittelt unter Verwendung von Sieben mit Siebmaschengrößen von 1000, 710, 500, 315, 200, 100, 50 und 32 µm und wobei 38 - 40 g Substanzmenge für 30 Minuten mit einer Amplitude von 1 mm im Intervall von 5 Sekunden gesiebt werden.
Ein entsprechendes Trägermaterial mit dieser Partikelgrößenverteilung weist darüber hinaus eine Schüttdichte im Bereich von 0,39 - 0,43 g/ml (nach DIN EN ISO 60), einen Schüttwinkel bzw. Fließneigungswinkel von 32,9 - 35,5° (nach DIN ISO 4324) und eine Stampfdichte von 0,52 - 0,57 g/ml (nach DIN EN ISO 787-11:1995) auf.
Insbesondere kann die Korngrößenverteilung dieses erfindungsgemäßen Trägermaterials so beschaffen sein, dass 13 - 15 Gew.-% des Granulats einen Partikeldurchmesser im Bereich von 50 bis 100 µm, 13 - 15 Gew.% des Granulats einen Partikeldurchmesser im Bereich von 100 - 200 µm, 15 - 23 Gew.-% des Granulats einen Partikeldurchmesser im Bereich von 200 - 315 µm und 46 - 50 Gew.-% des Granulats einen Partikeldurchmesser im Bereich von 315 - 500 µm aufweisen, wobei der Anteil <50 µm und der Anteile > 500 µm jeweils weniger als 6 Gew.-% beträgt.

Das erfindungsgemäße Trägermaterial ist vorzugsweise zu Tabletten mit einem Gewicht von 500 mg und einem Durchmesser von 11 mm verpressbar (instrumentierte Tablettiermaschine: KORSCH EK 0-DMS). Wenn es mit einer Preßkraft von 5 kN zu Tabletten verpresst wird, werden bevorzugt Produkte mit einer Härte von mind. 200 N erhalten.
Bei einer Preßkraft von 10 kN wird eine Tablettenhärte von vorzugsweise mindestens 340 N erhalten, wobei die hergestellten Tabletten eine Zerfallszeit von maximal 350 Sekunden aufweisen. Gegenstand der vorliegenden Erfindung sind also bevorzugt entsprechende Trägermaterialien mit der oben genannten Zusammensetzung von Sorbit und Xylit im Gewichtsverhältnis von 7 : 3 mit einem Wassergehalt < 1 Gew.% und einer BET-Oberfläche von 1,01 - 1,16 m²/g (nach DIN ISO 9277), sowie aus diesem Trägermaterial hergestellte Tabletten oder andere pharmazeutische Formulierungen mit oder ohne Wirkstoff.

Es wurde nun gefunden, dass diese gewünschten Eigenschaften durch die Co-Sprühgranulierung in der Wirbelschicht oder die Co-Sprühtrocknung einer Kombination, insbesondere bestehend aus 7 Gewichtsteilen Sorbit und 3 Gewichtsteilen Xylit, zu erzielen sind. Durch dieses Co-Sprühverfahren erhält man ein Material das bereits bei niedrigen Preßkräften Tabletten mit besonders hoher Härte liefert. Die erzielten Härten liegen deutlich höher als diejenigen von Materialien, bestehend aus einfachen physikalischen Mischungen der Einzelkomponenten - selbst in Xylit-Kombinationen mit sehr gut verpreßbarem DC-Sorbit. Gleichzeitig zeigt dieses erfindungsgemäße co-sprühgranulierte bzw. cosprühgetocknete Material bei vergleichbaren Tablettenhärten immer noch ausreichend schnelle Zerfallseigenschaften. Der Xylitanteil bewirkt darüber hinaus aufgrund seiner gegenüber Sorbit erhöhten Süße und seines Kühleffekts ein angenehmes Geschmacksempfinden in der Mundhöhle.

Insbesondere wurde gefunden, dass durch die Co-Sprühgranulierung in der Wirbelschicht einer solchen Sorbit/Xylit-Mischung ein Granulat mit Partikeldurchmessern im Bereich von 50 bis 500 µm erhalten wird, welches gegenüber üblichen co-gesprühten Materialien eine höhere Schüttdichte aufweist. So wurde durch Co-Sprühung einer Sorbit/Xylit-Lösung, worin die beiden Polyole in einem Gewichtsverhältnis von 7:3 enthalten waren, ein Material mit einer erhöhten Schüttdichte von 0,39 - 0,43 g/ml erhalten. Weiterhin weist dieses Material einen Fließneigungswinkel von 35,5-32,9° und eine Stampfdichte von 0,52 - 0,57g/ml auf. Das so erhaltene Material lässt sich gegenüber Materialien mit geringerem Schüttgewicht zu Tabletten mit einem erhöhten Gewicht bei gleich bleibenden Tablettendimensionen verpressen.

Materialien mit einer entsprechend erhöhten Schüttdichte können durch eine abgestimmte Anlagenfahrweise direkt aus der Co-Sprühgranulierung in der Wirbelschicht (kontinuierliche Fahrweise) oder aus der Co-Sprühtrocknung in einem Sprühtrocknungsturm erhalten werden. Es ist aber auch möglich, zur Einstellung der Schüttdichte das erhaltene Pulvermaterial, welches einen zu hohen Anteil an Partikeln mit einem Partikeldurchmesser größer als 500µm oder an grobkörnigem Material mit einem Durchmesser von >1000µm aufweist, zu sieben und die Partikelfraktion mit Partikeldurchmessem im Bereich von <500 µm abzutrennen und auf diese Weise ein Material mit einer geeigneten erhöhten Schüttdichte zu erhalten, welches es wiederum ermöglicht, Tabletten mit dem gewünschten erhöhten Gewicht -auch bei höheren Preßkräften- herzustellen. Diese Tabletten weisen darüber hinaus auch noch höhere Härten als Tabletten auf Basis von physikalische Mischungen aus DC-Sorbit und Xylit auf und lösen sich im Mund schnell auf, obwohl kein Binde- oder Sprengmittel zugesetzt worden ist.

Die durch Co-Sprühung in der Wirbelschicht oder durch Co-Sprühtrocknung granulierten Materialien zeigen gegenüber entsprechend zusammengesetzten physikalischen Mischungen der Einzelkomponenten über einen weiten Presskraft- und Tablettenhärtebereich ein deutlich verbessertes Presskraft/Härteprofil auf. Gleichzeitig zeigen mit diesen Materialien hergestellte Tabletten ein verhältnismäßig gutes Auflöseverhalten, und zwar ohne Zusatz von Sprengmitteln im Vergleich zu Tabletten, hergestellt aus entsprechenden physikalischen Mischungen von im Handel erhältlichen, pulverförmigen und kristallinen Xylit-Typen (C*Xylidex CR 16056, Xylisorb 90, Xylitab 300 und Xylisorb 300) mit dem per se sehr gut komprimierbaren, käuflichen DC-Sorbit Parteck SI 150 oder Parteck SI 400.

Mit dem erfindungsgemäßen Sorbit/Xylit Granulat ist es dem Galeniker nun möglich, die Entwicklung qualitativ hochwertige Lutsch-, Kau-und ODT-Formulierungen schnell durchzuführen. Durch die sehr guten Direkttablettierungseigenschaften wird zudem die industrielle Produktion vereinfacht und ökonomisch gestaltet.

Die Herstellung des erfindungsgemäßen Granulats kann in an sich dem Fachmann bekannter Weise in einer handelsüblichen Sprühgranulierungsanlage erfolgen. Bevorzugt erfolgt die Herstellung in einer Anlage mit Wirbelbett zur Nachtrocknung des Materials und Pulverrückführung durch eine kontinuierliche Fahrweise. Entsprechende Anlagen sind dem Fachmann bekannt, wie z. B. aus den Patentschriften EP 1 453 781 B1 und EP 1 319 644 B1 bzw. aus WO 00/76650 A1. Solche Anlagen erlauben es dem Fachmann durch gezielte Einstellung des Pulverbettes, des Gasstroms, der Temperaturführung und gegebenenfalls zusätzliches Aufsprühen von zusätzlicher Ausgangslösung, sowie einer gesteuerten Produktrückführung von feinem Material, gezielt die gewünschte Korngröße des Granulats einzustellen. Einfachere Anlagen finden sich in den Patentschriften EP 0738 252 B1 oder EP 0904059 B1.

Die Herstellung des sprühgranulierten Materials mit einem Gehalt an Sorbit und Xylit im Gewichtsverhältnis 7 : 3 kann erfolgen, indem
a) in einem ersten Schritt ein flüssiges Medium, Sprühgas, und gegebenenfalls vorgelegtes oder zurückgeführtes pulverförmiges Material und Heißluft zusammengeführt werden,
b) das entstehende pulverförmige Produkt in ein Wirbel- oder Fließbett fällt, aufgenommen, fluidisiert und weitertransportiert wird und gegebenenfalls
c) in einem oder mehreren Granulierungsschritt(en) mit weiterem flüssigen Medium besprüht, getrocknet
d) und im Fließbett in Richtung der Pulverdosieranlage gefördert wird, von welcher aus gegebenenfalls
e) eine Teilmenge pulverförmiges Material in den Prozeß zurückgeführt wird
   und
f) das getrocknete Granulat der Pulverdosieranlage zugeführt und aus der Anlage ausgetragen wird.

Durch eine gezielte Einstellung des im Wirbelbett von unten zugeführten Gasstroms ist es möglich, sowohl größere und schwerere Pulverpartikel als auch kleinere und leichtere Partikel aus dem gewünschten Produktstrom abzutrennen und in den Prozess über die Pulverrückführung zurückzuführen. Dabei kann in einer besonderen Variante dieses Verfahrens pulverförmiges Material vor der Rückführung zerkleinert werden, wodurch die erhaltene Korngröße eingestellt und reguliert werden kann. Bei optimaler Prozessführung ist es somit möglich, cosprühgranuliertes Material mit einem Partikeldurchmesser im Bereich von 50 bis 500 µm herzustellen.

Andernfalls ist es in einem nachgeschalteten Schritt möglich, aus dem erhaltenen Produkt das sogenannte Über- und Unterkorn mit zu geringer als auch zu hoher Partikelgröße durch Sieben abzutrennen.

Als Sprüh-, Träger- und Heizgas kann Luft oder ein Inertgas ausgewählt aus der Gruppe N2 und CO2 verwendet werden. Das Gas wird vorzugsweise im Kreislauf geführt, wobei es durch Filter oder speziell mit Hilfe von Dynamikfiltern von Partikeln befreit wird, im Kondensator getrocknet und erneut den Sprühdüsen zugeführt bzw. aufgeheizt und in das Fließbett eingeführt wird.

Erfindungsgemäß wird vorzugsweise als Edukt eine Polyolmischung von Sorbit und Xylit im Gewichtsverhältnis von 7 : 3 eingesetzt. Dieses Edukt wird als wässrige, 30 - 50%-ige Lösung eingesetzt und in die Anlage mit einer Temperatur im Bereich von 60 bis 95 °C versprüht. Bevorzugt wird eine 35 - 45%-ige Lösung eingesetzt. Besonders bevorzugt wird die Lösung vor dem Versprühen auf eine Temperatur im Bereich von 70 bis 85 °C, insbesondere von 75 bis 80°C, erwärmt.

Erfindungsgemäß können an verschiedenen Stellen der Anlage Lösungen mit unterschiedlichen Eduktkonzentrationen eingesetzt werden. So hat es sich als sinnvoll erwiesen, Sprühdüsen oberhalb des Fließbetts in Richtung des Produktaustrags mit Lösungen mit höheren Eduktkonzentrationen zu beschicken als Sprühdüsen, welche sich am Anfang des Fließbetts befinden. Es kann daher zum Ende des Fließbetts hin eine Lösung mit einer Eduktkonzentration von etwa 60 Gew.-% bezogen auf die Gesamtlösung eingesetzt werden, wohingegen die Zweistoffdüse mit Pulverrückführung bevorzugt mit einer etwa 30 - 50%-gen wässrigen Lösung betrieben wird. Auf diese Weise können die Produkteigenschaften nochmals im gewünschten Sinn beeinflusst werden, wobei bei dieser Fahrweise die Anlagenparameter genau zu beachten sind.

Durch Variation der Parameter, Sprühdruck, Flüssigkeitsmenge, zurückgeführte Pulvermenge, Heißluftstrom und Temperatur der Heißluft lässt sich gezielt die Partikelgröße im Bereich zwischen 50 und 500 µm einstellen. Die Trocknungsbedingungen sind so gewählt, dass der Wassergehalt des entnommenen Produktes <1 Gew.-% beträgt.

Zur Durchführung der Co-Sprühgranulierung können die Betriebsparameter einer kontinuierlich arbeitenden Anlage, bei der sich ein Sprühdüsensystem über einem Wirbelbett befindet, in welchem das gebildete Produkt zur Nachtrocknung und gegebenenfalls zur Nachgranulierung für eine bestimmte Strecke durch einen von unten eingeblasenen Luftstrom gefördert wird, folgendermaßen eingestellt werden, um ein gleichbleibendes Produkt zu erhalten:
Der Sprühdruck der Zweistoffdüsen ist im Bereich von 2 - 4 bar einzustellen. Vorzugsweise wird im Bereich von 2,5 bis 3,5 bar gearbeitet. Die der Zweistoffdüse zugeführte Menge Heißgas ist so zu regulieren, dass etwa 1,5 bis 3 m3/(h kg Lösung) mit einer Temperatur von etwa 80 bis 110 °C gefördert werden. Es wurde gefunden, dass bei höherer Heißgaszufuhr eine bessere Produktqualität erhalten wird, wenn mit niedrigerer Temperatur gearbeitet wird.
Die Pulverrückführung ist erfindungsgemäß so einzustellen, dass eine Feststoffrückführung im Bereich von 0,2 - 2,0 kg Feststoff/(h kg Lösung) erfolgt. Vorzugsweise wird im Bereich von 0,5 bis 1,5 kg Feststoff/(h kg Lösung) gearbeitet. Besonders günstig gestaltet sich der Prozess, wenn die Feststoffrückführung im Bereich von 0,5 bis 1,0 kg Feststoff/(h kg Lösung) liegt.
Zur Durchführung des Verfahrens muss in die Anlage vorgewärmte Luft eingespeist werden. Gute Ergebnisse werden erzielt, wenn die der Anlage zugeführte Luft auf eine Temperatur im Bereich von 45 - 120 °C vorgewärmt wird. Günstig ist es für den erfindungsgemäßen Prozess, wenn die Zuluft eine Temperatur im Bereich von 65 bis 110 °C aufweist. Besonders vorteilhaft für die Bildung eines gleichmäßigen Produkts mit guten Tablettiereigenschaften ist es, wenn die Temperatur der eingespeisten Zuluft im Bereich von 70 bis 100 °C liegt.
Die zugeführte Zuluftmenge ist erfindungsgemäß so zu regeln, dass 1000 - 2000 m3/m2 pro Stunde, insbesondere 1200 bis 1700 m3/m2 pro Stunde, in die Anlage eingespeist werden.

In Verbindung mit den übrigen eingestellten Parametern liegen besonders günstige Verfahrensbedingungen vor, wenn der Luftstrom in der Anlage so geführt wird, dass sich die Ablufttemperatur im Bereich von 30 -50 °C einstellt.

Weiterhin hat es sich als günstig herausgestellt, die Verfahrensbedingungen so zu regulieren, dass sich die im Wirbel- oder Fließbett befindliche Pulvermenge auf eine Bettmenge von 50 - 150 kg/m2 Bett einstellt. Besonders günstig ist es, wenn die Bettmenge im Bereich von 80 - 120 kg/m2 Bett liegt.

Es wurde auch gefunden, dass sich der Prozess insbesondere durch gezielte Rückführung eines Teils des gebildeten Pulvers mit ausgewählter Partikelgröße steuern lässt.

Insbesondere kann eine Pulverrückführung sowohl durch Pulverentnahme direkt aus dem Fließbett, bzw. aus dem Wirbelbett erfolgen als auch durch Rückführung einer sehr feinteiligen Pulverfraktion, welche bei der Konfektionierung, d. h. Homogenisierung der Partikelgröße durch Siebung und Abfüllung des hergestellten Produkts anfällt.

Darüber hinaus kann die Herstellung der erfindungsgemäßen Produkte auch durch Co-Sprühtrocknung in einfacher aufgebauten Sprühtürmen erfolgen, wie sie in den Patentschriften EP 0738 252 B1 oder EP 0904059 B1 und
DE 32 45 170 eingesetzt worden sind, und in denen sich die Sprüheinheit direkt über dem Wirbelbett befindet. Dabei ist jedoch wesentlich, dass durch die Co-Sprühtrocknung ein ausreichend großes Korn aufgebaut werden kann. Hierzu ist es vorteilhaft, wenn die Co-Sprühtrocknung in Gegenwart von rückgeführtem Produkt erfolgt und sich auf diese Weise ein vorteilhaft aufgebautes Granulat aufbauen kann mit entsprechender Korngröße und einem feinkristallin aufgebauten Korn. Um diese Kornstruktur in einfacher aufgebauten Anlagen zu erhalten, muss die Sprühtrocknung bei verhältnismäßig niedrigen Temperaturen durchgeführt werden, wobei die Luft vorzugsweise im Gegenstrom geführt wird. Weiterhin muss eine ausreichend lange Aufenthaltszeit im Wirbelbett gewährleistet sein, so dass dem Produkt ausreichend Feuchte entzogen werden kann; die Restfeuchte des Fertigproduktes sollte < 1 Gew.% betragen. Die Verwendung einer Zweistoffdüse, die es erlaubt, zurückgeführtes Pulver direkt in die Sprühzone einzuführen, ermöglicht es weiterhin, in einem Sprühturm mit anschließendem Wirbelbett die erfindungsgemäßen Produkte herzustellen, wenn die Gas-, Produkt- und Lösungsströme optimal zu einander eingestellt werden bei geeigneter Temperatur- und Drucksteuerung.

Die Herstellung des sprühgetrockneten Granulats mit einem Gehalt an Sorbit und Xylit im Gewichtsverhältnis 7 : 3 kann in einem solchen Sprühturm erfolgen, indem z.B. eine 40%ige wässrige Sorbit/ Xylit -Lösung bei einer Eingangstemperatur der Trocknungsluftluft von 120 - 180°C sprühgetrocknet wird, wobei gleichzeitig rückgeführtes Pulver des co-gesprühten Sorbit/Xylit 7 : 3 eingetragen wird, und die Ausgangstemperatur des Gas- und Produktstroms bei 60 - 70°C liegt. Zu Beginn des Sprühprozesses wird, falls kein cogesprühtes Sorbit/Xylit 7 : 3 aus vorhergehenden Sprühungen zur Verfügung steht, eine physikalische Mischung aus pulverförmigem Sorbit mit pulverförmigem Xylit im Gewichtsverhältnis 7 : 3 zur Rückführung bis zur Stabilisierung des Prozesses eingesetzt. Das Verhältnis von eingespeister Sprühlösung (bezogen auf Festsubstanz in der Lösung), eingespeister Pulverrückführung und der entnommenen Menge Fertigprodukt beträgt vorzugsweise pro Zeiteinheit etwa dem Verhältnis von 1 : 2 : 1. Die Sprühbedingungen -insbesondere die Luftmenge und das Verhältnis Zuluft/Ablufttemperatur - sind so zu justieren, dass der Wassergehalt im Endproduktes 1 Gew.% nicht übersteigt.

Wie bereits oben erwähnt werden beim Verpressen der erfindungsgemäßen Trägermaterialien (instrumentierte Tablettiermaschine: KORSCH EK 0-DMS) bei einer Preßkraft von 5 kN sehr gute Tablettenhärten erzielt, welche deutlich höher als 200N liegen, welche aber bei einer Presskraft von 10 kN nochmals auf >340N gesteigert werden. Das Material zeigt somit in einem bevorzugten Preßkraftbereich, welcher zur möglichsten Schonung der Tablettierwerkzeuge und Tablettiermaschinen gewählt werden sollte, bereits sehr gute Bindungseigenschaften. Die sehr gute mechanische Stabilität der hergestellten Produkte - gemessen als Friabilität - gewährleistet eine sichere Handhabung der erhaltenen Preßlinge. Gleichzeitig sind die durch Versuche ermittelten Zerfallszeiten der so hergestellten Tabletten im Vergleich zu solchen, hergestellt aus den physikalischen Mischungen, nur unwesentlich erhöht bzw. vergleichbar und dies bei gleichzeitig deutlich höheren Tablettenhärten. Auch ist es durch geeignete Wahl der eingestellten Parameter während der Co-Sprühgranulierung möglich, die Partikelgrößenverteilung des Trägermaterials so einzustellen, dass sich eine gewünschte Zerfallszeit einstellt. Wie beispielsweise in den im folgenden wiedergegebenen Tabellen zu sehen ist, weisen Produkte mit einer leicht veränderten Partikelgrößenverteilung nach dem Verpressen mit gleicher Preßkraft unterschiedliche Zerfallszeiten auf. Dementsprechend ist das erfindungsgemäße Material ausgezeichnet geeignet zur Herstellung von Formulierungen in Direkttablettierungsprözessen. Insbesondere ist es geeignet zur Herstellung von Tablettenformulierungen für in der Mundhöhle schnell Wirkstoff-freisetzende Tabletten.

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung.

Zum besseren Verständnis und zur Verdeutlichung der Erfindung werden im folgenden zwei Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten immer in °C.

### Beispiele

Eine Lösung aus 8 Teilen Wasser und 4 Teilen Feststoff (bestehend aus 70 Gew.-% Sorbit und 30 Gew.-% Xylit) wird in einem Sprühtrockner bzw. in einem Wirbelschichtgranulator, wie in der Patentschrift EP 1 453 781 B1 beschrieben, in eine Granulatfrom überführt. Da das Material an sich zum Kleben neigt, wird durch die Feststoffrückführung die Verarbeitbarkeit verbessert und auch das Kornspektrum in den gewünschten Bereich verschoben. Sofern dieses notwendig ist, wird durch einen nachgeschalteten Siebprozess die optimale Kornfraktion eingestellt. Durch die gezielte Einstellung der optimalen Kornfraktion kann die Schütt- und Stampfdichte zusätzlich eingestellt werden.

Zur Charakterisierung des Materials werden folgende analytischen Verfahren angewandt:
- Schüttdichte: gemäß DIN EN ISO 60
- Stampfdichte: gemäß DIN EN ISO 787-11: 1995
- Schüttwinkel: gemäß DIN ISO 4324
- Partikelgrößeverteilung:
   Trockensiebung über Siebturm Fa.Retsch (Deutschland) AS 200 control, g, ; Siebdurchmesser: 200 mm; Siebmaschengrößen: 1000, 710, 500, 315, 200, 100, 50 und 32 µm; Substanzmenge zur Siebung: 38-40g; Siebzeit: 30 Minuten; Amplitude: 1mm; Intervall: 5 Sekunden; Durchführung gem. Herstellerangaben (http://www.retsch.com/de/)
- Tablettierungsprüfung:
   495 g des auf seine Tablettierungseigenschaften zu prüfende Material werden mit 5g Parteck LUB MST (Magnesiumstearat pflanzlich) EMPROVE exp PhEur, BP, JP, NF, FCC Art.Nr. 1.00663 (MERCK KGaA, Deutschland) versetzt - das Magnesiumstearat wird zuvor über ein 250µm Sieb abgelegt - und 5 Minuten in einem verschlossenen Edelstahlbehälter [Fassungsvolumen: ca. 2 l, Höhe: ca.19,5 cm, Durchmesser: ca.12 cm (Außenmaße)] auf einem Labor-Taumelmischer (Turbula, Fa. Willy A. Bachofen (Schweiz) gemischt. Die Verpressung zu 500 mg Tabletten (11 mm Stempel, rund, flach, mit Facette) erfolgt auf einer instrumentierten Excenter-Tablettiermaschine Korsch EK 0-DMS (Fa. KORSCH, Deutschland) mit dem Auswertesystem Catman 5.0 der Fa. Hottinger Baldwin Messtechnik - HBM (Deutschland).

Je angewendeter Presskraft (5, 10, 20 und 30 kN; jeweils +/- 10%) werden mindestens 100 Tabletten zur Auswertung der galenischen Kennzahlen hergestellt.
- Tablettenhärte, Durchmesser und Höhe: Erweka TBH 30 MD; Fa. Erweka (Deutschland); Durchschnittsdaten aus jeweils 20 Tablettenmessungen pro Preßkraft
- Tablettenabrieb: Friabiliätsprüfgerät Fa.Erweka (Deutschland); Geräteparameter und Ausführung der Messungen gemäß Ph Eur 5. Ausgabe "Friabilität von nicht überzogenen Tabletten"
- Tablettenmasse: Durchschnittswert aus der Wägung von 20 Tabletten; Waage: Mettler AT 201 Fa.Mettler (Deutschland)
- Tablettenzerfall: automatisiertes Tablettenprüfgerät disi 4 der Fa.Biomation (Deutschland); Medium: entsalztes Wasser bei 37°C; Geräteparameter und Ausführung gemäß Ph Eur 5.Ausgabe "Zerfallszeit von Tabletten und Kapseln"
- BET Oberfläche: Auswertung und Durchführung gemäß Literatur "BET Surface Area by Nitrogen Absorption", S.Brunauer et al. (Journal of American Chemical Society, 60, 9, 1938) sowie DIN ISO 9277; Geräte: ASAP 2420 Fa.Micromeritics Instrument Corporation (USA); volumetrisches Verfahren; Stickstoff; Einwaage ca. 3g +/- 5% mit Probenvorbereitung (Ausheizung 3,0°C/Min. bis Zieltemperatur 50°C): 10 Stunden/50°C

Die Handhabung, Lagerung, Weiterverarbeitung und Prüfung der Granulate und der Preßlinge erfolgt bei Temperaturen von 20 - 25 °C und rel. Feuchten von 20-40%

**Charakterisierung: co-granuliertes Xylit/ Sorbit 3:7 (2 Beispiele)**

| | Erfindungsgemäßes Produkt (1.Beispiel) | | Erfindungsgemäßes Produkt (2.Beispiel) | |
|---|---|---|---|---|
| Schüttdichte | 0,39g/ml | | 0,43g/ml | |
| Fließneigungswinkel | 35,5° | | 32,9° | |
| Stampfdichte | 0,52g/ml | | 0,57g/ml | |
| Wassergehalt | 0,90 Gew.% | | 0,87Gew.% | |
| BET-Oberfläche | 1,01m²/g | | 1,16 m²/g | |
| Partikelverteilung (Gew.%) | <32µm: | 0 | <32µm: | 0,12 |
| | 32-50µm: | 2,72 | 32-50µm: | 5,47 |
| | 50-100µm: | 13,53 | 50-100µm: | 14,31 |
| | 100-200µm: | 13,32 | 100-200µm: | 13,70 |
| | 200-315 µm: | 22,97 | 200-315 µm: | 15,95 |
| | 315-500µm: | 46,33 | 315-500µm: | 49,44 |
| | >500m: | 1,13 | >500ym: | 1,01 |

**Tablettierungsdaten: co-granuliertes Xylit/ Sorbit im Gewichtsverhältnis von 3 : 7 (2 Beispiele) im Vergleich zu physikalischen Mischungen C*Xylidex CR 16056 (Cerestar) bzw. Xylisorb 90 (Roquette Freres) mit DC-Sorbit Parteck SI 150 bzw. 400 (Merck KGaA) im Gewichtsverhältnis von 3 : 7 (Xylit/Sorbit)**

| Produkt | Preßkraft | Tablettenhärte | Friabilität | Zerfall |
|---|---|---|---|---|
| | [kN] | [N] | [Gew.%] | [sec] |
| Erfindungsgemäßes Produkt | 5 | 264 | 0,09 | 338 |
| | 10 | 369 | 0,09 | 332 |
| 1. Beispiel | 20 | 389 | 0,13 | 319 |
| | 30 | 399 | 0,14 | 465 |
| Erfindungsgemäßes Produkt | 5 | 244 | 0,11 | 233 |
| | 10 | 352 | 0,11 | 265 |
| 2. Beispiel | 20 | 345 | 0,10 | 252 |
| | 30 | 394 | 0,12 | 536 |
| Physikalische Mischung: C*Xylidex CR 16056/Parteck SI150 3:7 | 5 | 108 | 0,25 | 239 |
| | 10 | 177 | 0,14 | 249 |
| | 20 | 205 | 0,14 | 284 |
| | 30 | 208 | 0,15 | 290 |
| Physikalische Mischung: C*Xylidex CR 16056/ Parteck SI400 3:7 | 5 | 83 | 0,49 | 167 |
| | 10 | 165 | 0,21 | 253 |
| | 20 | 216 | 0,17 | 246 |
| | 30 | 225 | 0,15 | 268 |
| Physikalische Mischung: Xylisorb 90/ Parteck SI150 3:7 | 5 | 110 | 0,30 | 143 |
| | 10 | 205 | 0,14 | 286 |
| | 20 | 232 | 0,13 | 239 |
| | 30 | 215 | 0,19 | 320 |
| Physikalische Mischung: Xylisorb 90/ Parteck SI400 3:7 | 5 | 85 | 0,71 | 208 |
| | 10 | 213 | 0,25 | 337 |
| | 20 | 213 | 0,26 | 331 |
| | 30 | 220 | 0,21 | 330 |

**Tablettierungsdaten: co-granuliertes Xylit/ Sorbit 3:7 (2 Beispiele) im Vergleich zu physikalischen Mischungen von Xylitab 300 (Danisco) bzw. Xylisorb 300 (Roquette Freres) mit DC-Sorbit Parteck SI 150 und SI 400 (Merck KGaA) jeweils im Gewichtsverhältnis von 3:7 (Xylit/Sorbit)**

| Produkt | Preßkraft [kN] | Tablettenhärte [N] | Friabilität [Gew.%] | Zerfall [sec] |
|---|---|---|---|---|
| Erfindungsgemäßes Produkt | 5 | 264 | 0,09 | 338 |
| | 10 | 369 | 0,09 | 332 |
| 1. Beispiel | 20 | 389 | 0,13 | 319 |
| | 30 | 399 | 0,14 | 465 |
| Erfindungsgemäßes Produkt | 5 | 244 | 0,11 | 233 |
| | 10 | 352 | 0,11 | 265 |
| 2. Beispiel | 20 | 345 | 0,10 | 252 |
| | 30 | 394 | 0,12 | 536 |
| Physikalische Mischung: Xylitab 300/ Parteck SI150 3 : 7 | 5 | 121 | 1,23 | 140 |
| | 10 | 187 | 0,10 | 279 |
| | 20 | 241 | 0,13 | 315 |
| | 30 | 264 | 0,15 | 343 |
| Physikalische Mischung: Xylitab 300/ Parteck SI400 3 : 7 | 5 | 78 | 0,69 | 159 |
| | 10 | 159 | 0,27 | 330 |
| | 20 | 228 | 0,18 | 290 |
| | 30 | 242 | 0,15 | 279 |
| Physikalische Mischung Xylisorb 300/ Parteck SI150 3 : 7 | 5 | 129 | 0,30 | 204 |
| | 10 | 193 | 0,18 | 236 |
| | 20 | 236 | 0,15 | 262 |
| | 30 | 224 | 0,15 | 265 |
| Physikalische Mischung: Xylisorb 300/ Parteck SI400 3 : 7 | 5 | 72 | 0,85 | 225 |
| | 10 | 145 | 0,35 | 285 |
| | 20 | 215 | 0,24 | 227 |
| | 30 | 236 | 0,21 | 252 |

Bereits bei einer Preßkraft von 5 kN werden sehr gute Tablettenhärten erzielt, welche deutlich höher als 200N sind, welche aber bei einer Presskraft von 10 kN nochmals auf >340N gesteigert werden. Das Material zeigt somit in einem bevorzugten Preßkraftbereich zur möglichsten Schonung der Tablettierwerkzeuge und Tablettiermaschinen bereits sehr gute Bindungseigenschaften. Die sehr gute mechanische Stabilität - gemessen als Friabilität - gewährleistet eine sichere Handhabung der Preßlinge. Gleichzeitig sind die Zerfallszeiten im Vergleich zu Tabletten, hergestellt aus den physikalischen Mischungen, nur unwesentlich erhöht bzw. vergleichbar und dies bei gleichzeitig deutlich höheren Tablettenhärten. Das erfindungsgemäße Material ist daher ausgezeichnet geeignet zur Herstellung von Formulierungen in Direkttablettierungsprozessen, insbesondere aber auch für in der Mundhöhle schnell Wirkstoff-freisetzende Tabletten.

## Patentansprüche

1. Trägermaterial zur Herstellung von wirkstoffhaltigen und/oder aromatisierten Tablettenformulierungen, bestehend aus einer cosprühgetrockneten oder kontinuierlich cosprühgranulierten Sorbit/Xylit-Mischung mit einem Wassergehalt < 1 Gew.-%, worin das Gewichtsverhältnis Sorbit zu Xylit 7 : 3 beträgt und mindestens 88 Gew.-% des Materials einen Partikeldurchmesser im Bereich von 50 - 500 µm aufweisen, und wobei der Anteil mit Durchmessern <50 µm und > 500 µm jeweils weniger als 6 Gew.-% beträgt, mit der Maßgabe, dass die Partikelgrößenverteilung durch Trockensiebung über einen Siebturm mit einem Siebdurchmesser von 200 mm ermittelt worden ist unter Verwendung von Sieben mit Siebmaschengrößen von 1000, 710, 500, 315, 200, 100, 50 und 32 µm, und wobei 38 - 40 g Substanzmenge für 30 Minuten mit einer Amplitude von 1 mm im Intervall von 5 Sekunden gesiebt worden ist.

2. Trägermaterial gemäß Anspruch 1 mit einer Schüttdichte von 0,39 - 0,43 g/ml (nach DIN EN ISO 60), aufweisend einen Schüttwinkel bzw. Fließneigungswinkel von 32,9 - 35,5° (nach DIN ISO 4324) und eine Stampfdichte von 0,52 - 0,57 g/ml (nach DIN EN ISO 787-11:1995).

3. Trägermaterial gemäß der Ansprüche 1 oder 2, **dadurch gekennzeichnet, dass** 13 - 15 Gew.-% des Granulats einen Partikeldurchmesser im Bereich von 50 bis 100 µm, 13 - 15 Gew.-% des Granulats einen Partikeldurchmesser im Bereich von 100-200 µm, 15 - 23 Gew.-% des Granulats einen Partikeldurchmesser im Bereich von 200 - 315 µm und 46 - 50 Gew.-% des Granulats einen Partikeldurchmesser im Bereich von 315 - 500 µm aufweisen, wobei der Anteil <50 µm und der Anteil > 500 µm jeweils weniger als 6 Gew.-% beträgt.

4. Direkt verpressbares Trägermaterial gemäß einem oder mehreren der Ansprüche 1 bis 3 mit einer BET-Oberfläche von 1,01 - 1,16 m²/g (nach DIN ISO 9277).

5. Tablette, hergestellt aus einem Trägermaterial gemäß einem oder mehreren der Ansprüche 1 bis 4, **dadurch gekennzeichnet, dass** eine Menge Trägermaterial mit einem Gewicht von 500 mg zu einer Tablette mit einem Durchmesser von 11 mm verpresst wird, wobei durch eine Presskraft von 5 kN eine Tabletten mit einer Härte von mindestens 200 N und einer Zerfallszeit von maximal 350 Sekunden generiert wird.

6. Tablette gemäß Anspruch 5, **dadurch gekennzeichnet, dass** durch eine Presskraft von 10 kN eine Tabletten mit einer Härte von mindestens 340 N und einer Zerfallszeit von maximal 350 Sekunden generiert wird.

7. Tabletten oder andere pharmazeutische Formulierungen mit oder ohne Wirkstoff, hergestellt unter Verwendung eines Trägermaterials gemäß einem oder mehreren der Ansprüche 1 - 3.

8. Wirkstoff- und/oder aromastoffhaltigen Tablettenformulierungen, hergestellt unter Verwendung eines Trägermaterials gemäß einem oder mehreren der Ansprüche 1 - 3.

9. Verwendung eines Trägermaterials gemäß einem oder mehreren der Ansprüche 1 - 3 zur Herstellung von Tablettenformulierungen, welche Vitamine, Mineralstoffe, Spurenelemente, funktionelle Lebensmittelbestandteile enthalten.

10. Verwendung eines Trägermaterials gemäß einem oder mehreren der Ansprüche 1 - 3 zur Herstellung von Tablettenformulierungen, welche Pflanzenbestandteile und Pflanzenextrakte enthalten.

11. Verwendung eines Trägermaterials gemäß einem oder mehreren der Ansprüche 1 - 3 zur Herstellung von Tablettenformulierungen, welche synthetische und natürliche Farbstoffe, natürliche und naturidentische Aromastoffe und andere geschmacksgebende Substanzen wie z.B. Süßstoffe aus der Gruppe Aspartam, Saccharin, Acesulfam K, Neohesperidin DC, Sucralose; Thaumatin und Steviosid, oder Fruchtaromen, Pfefferminzaromen, Menthol, Fruchtsäuren aus der Gruppe Citronensäure und Weinsäure oder geschmacksgebende Pflanzenauszüge enthalten.

12. Verwendung eines Trägermaterials gemäß einem oder mehreren der Ansprüche 1 - 3 zur Herstellung von Tablettenformulierungen, welche Substanzen mit pharmakologischer Wirkung wie z. B. Antacida, Antiinfektiva, auch zur lokalen Wirkung im Mund- und Rachenraum, Analgetika einschl. Opioide, Antiallergika, Antiemetika, Antidiarrhoika enthalten.

## Claims

1. Carrier material for the preparation of active-compound-containing and/or aromatised tablet formulations, consisting of a co-spray-dried or continuously co-spray-granulated sorbitol/xylitol mixture having a water content < 1% by weight, in which the sorbitol to xylitol weight ratio is 7 : 3 and at least 88% by weight of the material have a particle diameter in the range 50 - 500 µm, and where the proportion having diameters < 50 µm and > 500 µm is in each case less than 6% by weight, with the proviso that the particle size distribution has been determined by dry sieving through a sieve tower having a sieve diameter of 200 mm using sieves having sieve mesh sizes of 1000, 710, 500, 315, 200, 100, 50 and 32 µm, and where an amount of 38 - 40 g of substance has been sieved for 30 minutes with an amplitude of 1 mm in the interval of 5 seconds.

2. Carrier material according to Claim 1 having a bulk density of 0.39 - 0.43 g/ml (in accordance with DIN EN ISO 60), having an angle of repose of 32.9 - 35.5° (in accordance with DIN ISO 4324) and a tapped density of 0.52 - 0.57 g/ml (in accordance with DIN EN ISO 787-11:1995).

3. Carrier material according to Claim 1 or 2, **characterised in that** 13 - 15% by weight of the granules have a particle diameter in the range from 50 to 100 µm, 13 - 15% by weight of the granules have a particle diameter in the range 100-200 µm, 15 - 23% by weight of the granules have a particle diameter in the range 200 - 315 µm and 46 - 50% by weight of the granules have a particle diameter in the range 315 - 500 µm, where the proportion < 50 µm and the proportion > 500 µm is in each case less than 6% by weight.

4. Directly compressible carrier material according to one or more of Claims 1 to 3 having a BET surface area of 1.01 - 1.16 m²/g (in accordance with DIN ISO 9277).

5. Tablet produced from a carrier material according to one or more of Claims 1 to 4, **characterised in that** an amount of carrier material having a weight of 500 mg is compressed to give a tablet having a diameter of 11 mm, where a tablet having a hardness of at least 200 N and a maximum disintegration time of 350 seconds is generated by a pressing force of 5 kN.

6. Tablet according to Claim 5, **characterised in that** a tablet having a hardness of at least 340 N and a maximum disintegration time of 350 seconds is generated by a pressing force of 10 kN.

7. Tablets or other pharmaceutical formulations with or without active compound, produced using an carrier material according to one or more of Claims 1 - 3.

8. Tablet formulations comprising active compound and/or flavouring agent, produced using an carrier material according to one or more of Claims 1 - 3.

9. Use of an carrier material according to one or more of Claims 1 - 3 for the preparation of tablet formulations which comprise vitamins, mineral substances, trace elements, functional food constituents.

10. Use of an carrier material according to one or more of Claims 1 - 3 for the preparation of tablet formulations which comprise plant constituents and plant extracts.

11. Use of an carrier material according to one or more of Claims 1 - 3 for the preparation of tablet formulations which comprise synthetic and natural dyes, natural and nature-identical flavouring agents and other flavour-providing substances, such as, for example, sweeteners from the group aspartame, saccharine, acesulfame K, neohesperidin DC, sucralose, thaumatin and stevioside, or fruit flavours, peppermint flavours, menthol, fruit acids from the group citric acid and tartaric acid or flavour-providing plant extracts.

12. Use of an carrier material according to one or more of Claims 1 - 3 for the preparation of tablet formulations which comprise substances having a pharmacological action, such as, for example, antacids, antiinfectives, also for local action in the oral and pharyngeal area, analgesics, including opioids, anti-allergics, anti-emetics, anti-diarrhoeals.

## Revendications

1. Matériau véhicule pour la préparation de formulations de comprimés contenant un composé actif et/ou aromatisées, constitué par un mélange de sorbitol/xylitol séché par co-atomisation ou granulé par co-atomisation en continu présentant une teneur en eau < 1% en poids, dans lequel le rapport en poids sorbitol sur xylitol est de 7 : 3 et au moins 88% en poids du matériau présentent un diamètre de particule dans la plage de 50 - 500 µm, et dans lequel la proportion présentant des diamètres < 50 µm et > 500 µm est dans chaque cas inférieure à 6% en poids, étant entendu que la distribution des tailles des particules a été déterminée par tamisage à sec au travers d'une tour de tamisage qui présente un diamètre de tamisage de 200 mm en utilisant des tamis présentant des tailles de maille de tamis de 1000, 710, 500, 315, 200, 100, 50 et 32 µm, et dans lequel une quantité de 38 - 40 g de substance a été tamisée pendant 30 minutes selon une amplitude de 1 mm selon l'intervalle de 5 secondes.

2. Matériau véhicule selon la revendication 1, présentant une masse volumique apparente de 0,39 - 0,43 g/ml (conformément à DIN EN ISO 60), présentant un angle d'éboulement de 32,9 - 35,5° (conformément à DIN ISO 4324) et une densité après tassement de 0,52 - 0,57 g/ml (conformément à DIN EN ISO 787-11:1995).

3. Matériau véhicule selon la revendication 1 ou 2, **caractérisé en ce que** 13 - 15% en poids des granules présentent un diamètre de particules dans la plage de 50 à 100 µm, 13 - 15% en poids des granules présentent un diamètre de particules dans la plage de 100-200 µm, 15 - 23% en poids des granules présentent un diamètre de particules dans la plage de 200 - 315 µm et 46 - 50% en poids des granules présentent un diamètre de particules dans la plage de 315 - 500 µm, dans lequel la proportion < 50 µm et la proportion > 500 µm sont dans chaque cas inférieures à 6% en poids.

4. Matériau véhicule directement compressible selon une ou plusieurs des revendications 1 à 3 présentant une aire de surface BET de 1,01 - 1,16 m²/g (conformément à DIN ISO 9277).

5. Comprimé produit à partir d'un matériau véhicule selon une ou plusieurs des revendications 1 à 4, **caractérisé en ce qu'**une quantité de matériau véhicule présentant un poids de 500 mg est comprimée de manière à obtenir un comprimé présentant un diamètre de 11 mm, dans lequel un comprimé présentant une dureté d'au moins 200 N et un temps de désintégration maximum de 350 secondes est généré au moyen d'une force de pression de 5 kN.

6. Comprimé selon la revendication 5, **caractérisé en ce qu'**un comprimé qui présente une dureté d'au moins 340 N et un temps de désintégration maximum de 350 secondes est généré au moyen d'une force de pression de 10 kN.

7. Comprimés ou autres formulations pharmaceutiques avec ou sans composé actif, produit(e)s en utilisant un matériau véhicule selon une ou plusieurs des revendications 1 - 3.

8. Formulations de comprimés comprenant un composé actif et/ou un agent aromatisant, produites en utilisant un matériau véhicule selon une ou plusieurs des revendications 1 - 3.

9. Utilisation d'un matériau véhicule selon une ou plusieurs des revendications 1 - 3 pour la préparation de formulations de comprimés qui comprennent des vitamines, des substances minérales, des oligo-éléments, des constituants alimentaires fonctionnels.

10. Utilisation d'un matériau véhicule selon une ou plusieurs des revendications 1 - 3 pour la préparation de formulations de comprimés qui comprennent des constituants de plantes et des extraits de plantes.

11. Utilisation d'un matériau véhicule selon une ou plusieurs des revendications 1 - 3 pour la préparation de formulations de comprimés qui comprennent des colorants synthétiques et naturels, des agents aromatisants naturels et équivalents et d'autres substances génératrices d'arôme, telles que, par exemple, les édulcorants issus du groupe constitué par l'aspartame, la saccharine, l'acésulfame K, la néohespéridine DC, le sucralose, la thaumatine et la stévioside, ou les arômes de fruits, les arômes de menthe poivrée, le menthol, les acides de fruits issus du groupe constitué par l'acide citrique et l'acide tartarique ou les extraits de plantes générateurs d'arômes.

12. Utilisation d'un matériau véhicule selon une ou plusieurs des revendications 1 - 3 pour la préparation de formulations de comprimés qui comprennent des substances présentant une action pharmacologique, telles que, par exemple, les anti-acides, les anti-infectieux, également pour une action locale au niveau de la sphère orale et pharyngée, les analgésiques, incluant les opioïdes, les antiallergiques, les antiémétiques, et les anti-diarrhéiques.
